# EUROPEAN PATENT APPLICATION

(11) **EP 1 757 681 A1**
(43) Date of publication of application: **28.02.2007**
(21) Application number: 06023229.5
(22) Date of filing: 21.11.2001
(51) Int. Cl.: C12N 5/08, A61K 35/12, A61P 37/00

(54) **Tolerizing allografts of pluripotent stem cells**

(30) Priority: 22.11.2000 US 252688 P
(62) Divisional of application: 01987036.9
(71) Applicant: GERON CORPORATION, Menlo Park, CA 94025 (US)
(72) Inventor: Chiu, Choy-Pik, Cupertino, CA 95014 (US); Kay, Robert M., San Francisco, CA 94115 (US)
(74) Representative: Bassil, Nicholas Charles

(57) **Abstract**

This disclosure provides a system for overcoming HLA mismatch between an allograft derived from stem cells, and a patient being treated for tissue regeneration. A state of specific immune tolerance is induced in the patient, by administering a population of tolerizing cells derived from the stem cells. This allows the patient to accept an allograft of differentiated cells derived from the same source. This invention is important because it allows a single line o stem cells to act as a universal donor source for tissue regeneration in any patient, regardless.

## Description

### TECHNICAL FIELD

This invention relates generally to the fields of cell biology of embryonic cells, and transplantation immunology. More specifically, it describes a technology for creating specific immunotolerance in a patient so that they will accept an allograft made from pluripotent stem cells.

### REFERENCE TO RELATED APPLICATIONS

This application claims priority benefit of U.S. provisional patent application 60/252,688, filed November 22, 2000, pending. For purposes of prosecution of this application in the U.S., the priority application is hereby incorporated herein by reference in its entirety.

### BACKGROUND

Precursor cells have become a central interest in medical research. Many tissues in the body have a back-up reservoir of precursors that can replace cells that are senescent or damaged by injury or disease.

U.S. Patent 5,750,397 (Tsukamoto et al., Systemix) reports isolation and growth of human hematopoietic stem cells which are Thy-1+, CD34+, and capable of differentiation into lymphoid, erythroid, and myelomonocytic lineages. U.S. Patent 5,736,396 (Bruder et al.) reports methods for lineage-directed differentiation of isolated human mesenchymal stem cells, using an appropriate bioactive factor. The derived cells can then be introduced into a host for mesenchymal tissue regeneration or repair.

U.S. Patent 5,716,411 (Orgill et al.) proposes regenerating skin at the site of a burn or wound, using an epithelial autograft. U.S. Patent 5,766,948 (F. Gage) reports a method for producing neuroblasts from animal brain tissue. U.S. Patent 5,672,499 (Anderson et al.) reports obtaining neural crest stem cells from embryonic tissue. U.S. Patent 5,851,832 (Weiss et al., Neurospheres) reports isolation of putative neural stem cells from 8-12 week old human fetuses. U.S. Patent 5,968,829 (M. Carpenter) reports human neural stem cells derived from adult primary central nervous system tissue.

U.S. Patent 5,082,670 (F. Gage) reports a method for grafting genetically modified cells to treat defects, disease or damage of the central nervous system. Auerbach et al. (Eur. J. Neurosci. 12:1696, 2000) report that multipotential CNS cells implanted into animal brains form electrically active and functionally connected neurons. Brustle et al. (Science 285:754, 1999) report that precursor cells derived from embryonic stem cells interact with host neurons and efficiently myelinate axons in the brain and spinal cord.

Considerable interest has been generated by the development of embryonic stem cells, which are thought to have the potential to differentiate into almost any cell type. Until recently, the only mammal from which embryonic stem cells had been Isolated was the mouse. Thomson et al. recently isolated and propagated pluripotent stem cells from lower primates (U.S. Patent 5,843,780; Proc. Natl. Acad. Sci. USA 92:7844, 1995; Biol. Reprod. 5:254, 1996), and then from humans (Science 282:114, 1998). Gearhart and coworkers derived human embryonic germ (hEG) cell lines from fetal gonadal tissue (Shamblott et al., Proc. Natl. Acad. Sci. USA 95:13726, 1998; and U.S. Patent 6,090,622). International Patent Publication WO 99/20741 (Geron Corp.) refers to methods and materials for growing primate-derived primordial stem cells.

Both hES and hEG cells have the long-sought characteristics of pluripotent stem cells: they are capable of being grown in vitro without differentiating, they have a normal karyotype, and they remain capable of producing a number of different cell types. Clonally derived human embryonic stem cell lines maintain pluripotency and proliferative potential for prolonged periods in culture (Amit et al., Dev. Biol. 227:271, 2000).

Stem cells hold considerable promise for use in human therapy, acting as a reservoir for regeneration of almost any tissue compromised by genetic abnormality, trauma, or a disease condition.

### SUMMARY OF THE INVENTION

This disclosure provides a system that allows a single line of stem cells to act as a universal donor source for tissue regeneration in any patient, regardless of tissue type. HLA mismatch between the stem cell source and the patient is overcome by treating the patient with tolerizing cells derived from the stem cells. This allows the patient to undergo tissue regeneration using differentiated cells derived from the same source.

One aspect of the invention is a method for preparing cells for therapeutic use, comprising differentiating human pluripotent stem (hPS) cells into a first and second cell population, whereupon administration of the first population to an individual renders them immunotolerant to the second cell population.

The first cell population is MHC compatible with the second population, which means that the cells share at least one haplotype at the HLA-A and HLA-B loci. In a preferred embodiment, the cells in the two populations are autogenic - which can be attained by differentiating both populations from the same hPS cell line.

Particular types of tolerizing cells in the first cell population can have particular phenotypic or functional characteristics described in the sections that follow. The second cell population comprises cells of any type needed for tissue regeneration by the patient being treated.

Another aspect of the invention is a method for preparing a first cell population that renders an individual to whom it is administered immunotolerant to a second cell population, as already described.

Another aspect of the invention is a method of reconstituting cellular function in an individual, by administering the first and second cell population, as already described.

Another aspect of the invention is the use of a first cell population and a second cell population as already described for the preparation of pharmaceutical compositions. Included is a combination of pharmaceutical compounds, offered in kit form or distributed separately. Components of the combination are a first cell population that has been differentiated from human pluripotent stem (hPS) cells into a phenotype that renders a subject to whom it is administered immunotolerant to a second cell population; and a second cell population that is MHC compatible with the first cell population, as already described.

Other embodiments of the invention will be apparent from the description that follows.

### DETAILED DESCRIPTION OF THE INVENTION

Stem cell technology is being developed in the direction of creating banks of stem cells and their derivatives for tissue regeneration. The present invention recognizes that an important issue to be resolved is the histocompatibility mismatch between the cells of the graft and the patient. Stem cells and the cells differentiated from them are believed to express MHC antigens. Allografts of such cells are predicted to be the subject of hyperacute, acute, and chronic tissue rejection in the absence of immunosuppressive agents.

This invention solves compatibility of the stem cell allograft by inducing a state of immune tolerance that is cell specific. The patient is prepared by infusing with tolerizing cells that induce specific immunological unresponsiveness against the tissue type used in the allograft.

Tolerance induction is believed to include an adaptation of the immune system of the host - involving elimination or anergy of allospecific host lymphocytes, by interacting with MHC Class II presenting cells or other components of the tolerizing cell population. The host may also adopt new immune components from the tolerizing population (such as allospecific supressor or veto cells) - detectable as cellular chimerism in the host. These mechanisms are presented here to enhance the reader's appreciation of the Invention. It is not necessary that these mechanisms be understood or proved for the invention to be put into practice.

The invention takes advantage of a unique property of stem cells that allows the same line to be differentiated into both the tolerizing cell population, and other terminally differentiated cells (such as neural or hepatocyte precursors) used for tissue regeneration. Because of their high replicative capacity, the stem cells can be grown and differentiated to the quantity required for treatment. Administration of the tolerizing cells induces immunological anergy in the patient not only against Class I and Class II alloantigens, but also against the myriad of minor histocompatibility antigens and allotypic differences that may be present in the transplant.

The strategy described in this disclosure provides enormous potential for the use of stem cells in regenerative medicine. Faced with histocompatibility mismatch between stem cells and cells of a patient needing treatment, the clinician has previously been faced with difficult options - such as maintaining an enormous bank of pluripotent stem cells to have allotypes compatible with each patient - or else, subjecting patients to a severe regimen of immunosuppressive therapy until the graft is accepted.

With the present invention, a single line of stem cells can be used to tolerize any patient, and then to regenerate tissue in a manner that will be accepted by the patient's immune system.

### Definitions

Prototype "primate Pluripotent Stem cells" (pPS cells) are pluripotent cells derived from pre-embryonic, embryonic, or fetal tissue at any time after fertilization, and have the characteristic of being capable under appropriate conditions of producing progeny of several different cell types that are derivatives of all of the three germinal layers (endoderm, mesoderm, and ectoderm), according to a standard art-accepted test, such as the ability to form a teratoma in 8-12 week old SCID mice.

Included in the definition of pPS cells are embryonic cells of various types, exemplified by human embryonic stem (hES) cells, described by Thomson et al. (Science 282:1145, 1998); embryonic stem cells from other primates, such as Rhesus stem cells (Thomson et al., Proc. Natl. Acad. Sci. USA 92:7844, 1995), marmoset stem cells (Thomson et al., Biol. Reprod. 55:254, 1996) and human embryonic germ (hEG) cells (Shamblott et al., Proc. Natl. Acad. Sci. USA 95:13726, 1998). Other types of pluripotent cells are also included in the term. Any cells of primate origin that are capable of producing progeny that are derivatives of all three germinal layers are included, regardless of whether they were derived from embryonic tissue, fetal tissue, or other sources. Included are the human equivalents of early primitive ectoderm-like (EPL) cells (WO 99/53021 & WO 01/51611, Bresagen Ltd.). Also included are embryonal carcinoma (EC) cells (Pera et al., Int. J. Cancer 40:334, 1987), although it is generally preferable to use cells with a normal karyotype and not derived from a malignant source.

pPS cell cultures are described as "undifferentiated" when a substantial proportion of stem cells and their derivatives in the population display morphological characteristics of undifferentiated cells, clearly distinguishing them from differentiated cells of embryo or adult origin. Undifferentiated pPS cells are easily recognized by those skilled in the art, and typically appear in the two dimensions of a microscopic view in colonies of cells with high nuclear/cytoplasmic ratios and prominent nucleoli. It is understood that colonies of undifferentiated cells within the population will often be surrounded by neighboring cells that are differentiated. Nevertheless, the undifferentiated colonies persist when the population is cultured or, passaged under appropriate conditions, and individual undifferentiated cells constitute a substantial proportion (>20%, preferably >60%) of the cell population.

"Feeder cells" or "feeders" are terms used to describe cells of one type that are co-cultured with cells of another type, to provide an environment in which the cells of the second type can grow. The feeder cells are optionally from a different species as the cells they are supporting. For example, certain types of pPS cells can be supported by primary mouse embryonic fibroblasts, immortalized mouse embryonic fibroblasts, or human fibroblast-like cells differentiated from hES cells, as described later in this disclosure. pPS cell populations are said to be "essentially free" of feeder cells if the cells have been grown through at least one round after splitting in which fresh feeder cells are not added to support the growth of the pPS. Cultures essentially free of feeder cells contain less than about 5% feeder cells. Whenever a culture or cell population Is referred to In this disclosure as "feeder-free", what is meant is that the composition is essentially free of feeder cells according to the preceding definition, subject only to further constraints explicitly required.

The term "embryoid bodies" is a term of art synonymous with "aggregate bodies". The terms refer to aggregates of differentiated and undifferentiated cells that appear when pPS cells overgrow in monolayer cultures, or are maintained in suspension cultures. Embryoid bodies are a mixture of different cell types, typically from several germ layers, distinguishable by morphological criteria.

The terms "committed precursor cells", "lineage restricted precursor cells" and "restricted developmental lineage cells" all refer to cells that are capable of proliferating and differentiating into several different cell types, with a range that is typically more limited than pluripotent stem cells of embryonic origin capable of giving rise to progeny of all three germ layers. Non-limiting examples of committed precursor cells include hematopoietic lineage cells, described below; hepatocyte progenitors, which are pluripotent for bile duct epithelial cells and hepatocytes; and mesenchymal stem cells. Another example is neural restricted cells, which can generate glial cell precursors that progress to oligodendrocytes and astrocytes, and neuronal precursors that progress to neurons.

For the purposes of this description, the term "stem cell" can refer to either a pluripotent stem cell, or a committed precursor cell, both as defined above. Minimally, a stem cell has the ability to proliferate and form cells of more than one phenotype, and is also capable of self renewal - either as part of the same culture, or when cultured under different conditions. A stem cell can be identified as positive for the enzyme telomerase.

The terms "hematopoetic cell" and "hematopoietic lineage cell" are used interchangeably in this disclosure to refer to types of blood cells, including red cells, lymphocytes, monocytes, dendritic cells, eosinophils, basophils, and polymorphonuclear leukocytes. Included are non-circulating functional counterparts of these cells, such as erythroblasts in the bone marrow, lymphocytes compartmentalized in the lymph nodes or spleen, macrophages localized in the skin or the liver. Also included are precursor cells committed to differentiate into progeny having characteristic features of this lineage. The term is used in the description that follows for illustrative purposes.

Specific immunological "tolerance" is a state in which an individual mounts less of an immune response against a certain foreign substance as it does against other substances of a similar kind. In the context of this invention, immunological tolerance is especially desired against an allograft used for tissue regeneration. When the patient is specifically tolerized according to the invention, there is less of an immune response against the allograft than would otherwise result. Tolerance can be determined by measuring specific antibody, CTL, or T helper/inducer reactivity against the specific tissue, as described below, and compared with the reactivity before treatment, or in comparison with similar tissue of a different allotype.

Except were explicitly stated, there is no intention to limit the claimed invention to tolerizing cells of a particular phenotype. What is meant by a "tolerizing cell" is simply a cell which (upon administration to a subject) can induce specific immunological tolerance, as described above. There are many cell populations differentiated from pluripotent stem cells that have the toleragenic properties suitable for use in this invention, some of which will demonstrate morphological characteristics or markers of mesenchymal cells, or hematopoietic lineage cells.

Except where explicitly stated, there is not intention to limit the claimed invention to a specific mechanism of immune tolerance. Mechanisms may include but are not limited to depletion of B or T cells of a particular specificity, B or T cell anergy or unresponsiveness, or active suppression by suppressor T cells or veto cells. It is the resulting effect of tolerance that is of interest, which can be tested as described elsewhere in this disclosure.

### General Techniques

For further elaboration of general techniques useful in the practice of this invention, the practitioner can refer to standard textbooks and reviews in cell biology, tissue culture, and embryology. Included are Teratocarcinomas and Embryonic Stem Cells: A Practical Approach (E.J. Robertson, ed., IRL Press Ltd. 1987); Guide to Techniques in Mouse Development (P.M. Wasserman et al., eds., Academic Press 1993); Embryonic Stem Cell Differentiation in Vitro (M.V. Wiles, Meth. Enzymol. 225:900, 1993); Properties and uses of Embryonic Stem Cells: Prospects for Application to Human Biology and Gene Therapy (P.D. Rathjen et al., Reprod. Fertil. Dev. 10:31, 1998). Differentiation of stem cells is reviewed in Robertson (Meth. Cell Biol. 75:173, 1997); and Pedersen (Reprod. Fertil. Dev. 10:31, 1998).

For topics related to hematopoietic cell lines and Immunotolerance, the following publications are available: Hemopoietic Lineages in Health and Disease (N.G. Testa et al., eds., Marcel Dekker 1999); Immune Tolerance (J. Banchereau et al., Editions Scientifiques et Medicales Elsevier, 1996); and Immunological Tolerance (G. Bock et al. eds., John Wiley & Son Ltd, 1998).

### Sources of Pluripotent Stem Cells

The invention can be practiced using stem cells of any vertebrate species. Included are stem cells from humans; as well as non-human primates, domestic animals, livestock, and other non-human mammals. Amongst the stem cells suitable for use in this invention are primate pluripotent stem (pPS) cells derived from tissue formed after gestation, such as a blastocyst, or fetal or embryonic tissue taken any time during gestation. Non-limiting examples are primary cultures or established lines of embryonic stem cells or embryonic germ cells.

### Embryonic Stem Cells

Embryonic stem cells can be isolated from blastocysts of members of the primate species (Thomson et al., Proc. Natl. Acad. Sci. USA 92:7844, 1995). Human embryonic stem (hES) cells can be prepared from human blastocyst cells using the techniques described by Thomson et al. (U.S. Patent 5,843,780; Science 282:1145, 1998; Curr. Top. Dev. Biol. 38:133 ff., 1998) and Reubinoff et al, Nature Biotech. 18:399,2000.

Briefly, human blastocysts are obtained from human in vivo preimplantation embryos. Alternatively, in vitro fertilized (IVF) embryos can be used, or one-cell human embryos can be expanded to the blastocyst stage (Bongso et al., Hum Reprod 4: 706, 1989). Embryos are cultured to the blastocyst stage in G1.2 and G2.2 medium (Gardner et al., Fertil. Steril. 69:84, 1998). The zona pellucida is removed from developed blastocysts by brief exposure to pronase (Sigma). The inner cell masses are isolated by immunosurgery, in which blastocysts are exposed to a 1:50 dilution of rabbit anti-human spleen cell antiserum for 30 min, then washed for 5 min three times in DMEM, and exposed to a 1:5 dilution of Guinea pig complement (Gibco) for 3 min (Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers.

After 9 to 15 days, inner cell mass-derived outgrowths are dissociated into clumps, either by exposure to calcium and magnesium-free phosphate-buffered saline (PBS) with 1 mM EDTA, by exposure to dispase or trypsin, or by mechanical dissociation with a micropipette; and then replated on mEF in fresh medium. Growing colonies having undifferentiated morphology are individually selected by micropipette, mechanically dissociated into clumps, and replated. ES-like morphology is characterized as compact colonies with apparently high nucleus to cytoplasm ratio and prominent nucleoli. Resulting ES cells are then routinely split every 1-2 weeks by brief trypsinization, exposure to Dulbecco's PBS (containing 2 mM EDTA), exposure to type IV collagenase (-200 U/mL; Gibco) or by selection of individual colonies by micropipette. Clump sizes of about 50 to 100 cells are optimal.

### Embryonic Germ Cells

Human Embryonic Germ (hEG) cells can be prepared from primordial germ cells present in human fetal material taken about 8-11 weeks after the last menstrual period. Suitable preparation methods are described in Shamblott et al., Proc. Natl. Acad. Sci. USA 95:13726, 1998 and U.S. Patent 6,090,622.

Briefly, genital ridges are rinsed with isotonic buffer, then placed into 0.1mL 0.05% trypsin/0.53 mM sodium EDTA solution (BRL) and cut into <1 mm³ chunks. The tissue is then pipetted through a 100 *µ*L tip to further disaggregate the cells. It is incubated at 37°C for ~5 min, then ~3.5 mL EG growth medium is added. EG growth medium is DMEM, 4500 mg/L D-glucose, 2200 mg/L mM NaHCO₃; 15% ES qualified fetal calf serum (BRL); 2 mM glutamine (BRL); 1 mM sodium pyruvate (BRL); 1000-2000 U/mL human recombinant leukemia inhibitory factor (LIF, Genzyme); 1-2 ng/ml human recombinant bFGF (Genzyme); and 10 µM forskolin (in 10% DMSO). In an alternative approach, EG cells are isolated using hyaluronidase/collagenase/DNAse. Gonadal anlagen or genital ridges with mesenteries are dissected from fetal material, the genital ridges are rinsed in PBS, then placed in 0.1 ml HCD digestion solution (0.01 % hyaluronidase type V, 0.002% DNAse 1, 0.1% collagenase type IV, all from Sigma prepared in EG growth medium). Tissue is minced, incubated 1 h or overnight at 37°C, resuspended in 1-3 mL of EG growth medium, and plated onto a feeder layer.

Ninety-six well tissue culture plates are prepared with a sub-confluent layer of feeder cells (e.g., STO cells, ATCC No. CRL 1503) cultured for 3 days in modified EG growth medium free of LIF, bFGF or forskolin, inactivated with 5000 rad γ-irradiation. ~0.2 mL of primary germ cell (PGC) suspension is added to each of the wells. The first passage is done after 7-10 days in EG growth medium, transferring each well to one well of a 24-well culture dish previously prepared with irradiated STO mouse fibroblasts. The cells are cultured with daily replacement of medium until cell morphology consistent with EG cells is observed, typically after 7-30 days or 1-4 passages.

### Propagation of pPS Cells in an Undifferentiated State

pPS cells can be propagated continuously in culture, using culture conditions that promote proliferation without promoting differentiation. Exemplary serum-containing ES medium is made with 80% DMEM (such as Knock-Out DMEM, Gibco), 20% of either defined fetal bovine serum (FBS, Hyclone) or serum replacement (WO 98/30679), 1% non-essential amino acids, 1 mM L-glutamine, and 0.1 mM β-mercaptoethanol. Just before use, human bFGF is added to 4 ng/mL (WO 99/20741, Geron Corp.).

Traditionally, ES cells are cultured on a layer of feeder cells, typically fibroblasts derived from embryonic or fetal tissue. Embryos are harvested from a CF1 mouse at 13 days of pregnancy, transferred to 2 mL trypsin/EDTA, finely minced, and incubated 5 min at 37°C. 10% FBS is added, debris is allowed to settle, and the cells are propagated in 90% DMEM , 10% FBS, and 2 mM glutamine. To prepare a feeder cell layer, cells are irradiated to inhibit proliferation but permit synthesis of factors that support ES cells (~4000 rads γ-irradiation). Culture plates are coated with 0.5% gelatin overnight, plated with 375,000 irradiated mEFs per well, and used 5 h to 4 days after plating. The medium is replaced with fresh h\ES medium just before seeding pPS cells.

Scientists at Geron have discovered that pPS cells can alternatively be maintained in an undifferentiated state even without feeder cells. The environment for feeder-free cultures includes a suitable culture substrate, particularly an extracellular matrix such as Matrigel® or laminin. The pPS cells are plated at >15,000 cells cm⁻² (optimally 90,000 cm⁻² to 170,000 cm⁻²). Typically, enzymatic digestion is halted before cells become completely dispersed (say, ~5 min with collagenase IV). Clumps of ~10-2000 cells are then plated directly onto the substrate without further dispersal.

Feeder-free cultures are supported by a nutrient medium typically conditioned by culturing irradiated primary mouse embryonic fibroblasts, telomerized mouse fibroblasts, or fibroblast-like cells derived from pPS cells. Medium can be conditioned by plating the feeders at a density of ~5-6 × 10⁴ cm⁻² in a serum free medium such as KO DMEM supplemented with 20% serum replacement and 4 ng/mL bFGF. Medium that has been conditioned for 1-2 days is supplemented with further bFGF, and used to support pPS cell culture for 1-2 days.

Under the microscope, ES cells appear with high nuclear/cytoplasmic ratios, prominent nucleoli, and compact colony formation with poorly discernable cell junctions. Primate ES cells express stage-specific embryonic antigens (SSEA) 3 and 4, and markers detectable using antibodies designated Tra-1-60 and Tra-1-81 (Thomson et al., Science 282:1145, 1998). Mouse ES cells can be used as a positive control for SSEA-1, and as a negative control for SSEA-4, Tra-1-60, and Tra-1-81. SSEA-4 is consistently present on human embryonal carcinoma (hEC) cells. Differentiation of pPS cells in vitro results in the loss of SSEA-4, Tra-1-60, and Tra-1-81 expression and increased expression of SSEA-1. SSEA-1 is also found on hEG cells.

### Differentiating pPS Cells for Tissue Regeneration

Differentiation of the pPS can be initiated by first forming embryoid bodies. General principles in culturing embryoid bodies are reported in O'Shea, Anat. Rec. (New Anat. 257:323, 1999). pPS cells are cultured in a manner that permits aggregates to form, for example, by overgrowth of a pPS cell culture. Alternatively, pPS cells are harvested by brief collagenase digestion, dissociated into clusters, and plated in non-adherent cell culture plates. The aggregates are fed every few days, and then harvested after a suitable period, typically 4-8 days. The cells can then be cultured with factors or on a substrate that promotes enrichment of cells of a particular lineage. Embryoid bodies comprise a heterogeneous cell population, potentially having an endoderm exterior, and a mesoderm and ectoderm interior.

Scientists at Geron Corporation have discovered that pPS cells can be differentiated into committed precursor cells or terminally differentiated cells without forming embryoid bodies or aggregates as an intermediate step. Briefly, a suspension of undifferentiated pPS cells is prepared, and then plated onto a solid surface that promotes differentiation. Suitable substrates include glass or plastic surfaces that are adherent, for example, by coating with a polycationic substance such as poly-lysine. The cells are then cultured in a suitable nutrient medium that is adapted to promote differentiation towards the desired cell lineage.

In some circumstances, differentiation is further promoted by withdrawing serum or serum replacement from the culture medium, or by withdrawing a medium component that inhibits differentiation (e.g., bFGF). Differentiation can also be promoted by adding a medium component that promotes differentiation towards the desired cell lineage, or inhibits the growth of cells with undesired characteristics. For example, to generate cells committed to neural or glial lineages, the medium can include any of the following factors or medium constituents in an effective combination: Brain derived neurotrophic factor (BDNF), neutrotrophin-3 (NT-3), NT-4, epidermal growth factor (EGF), ciliary neurotrophic factor (CNTF), nerve growth factor (NGF), retinoic acid (RA), sonic hedgehog, FGF-8, ascorbic acid, forskolin, fetal bovine serum (FBS), and bone morphogenic proteins (BMPs).

General principals for obtaining tissue cells from pluripotent stem cells are reviewed in Pedersen (Reprod. Fertil. Dev. 6:543, 1994), and U.S. Patent 6,090,622. Other publications of interest include the following: For neural progenitors, neural restrictive cells and glial cell precursors, see Bain et al., Biochem. Biophys. Res. Commun. 200:1252, 1994; Trojanowski et al., Exp. Neurol. 144:92, 1997; Wojcik et al., Proc. Natl. Acad. Sci. USA 90:1305-130; and U.S. Patents 5,851,832, 5,928,947, 5,766,948, and 5,849,553. For cardiac muscle and cardiomyocytes see Chen et al., Dev. Dynamics 197:217, 1993 and Wobus et al., Differentiation 48:173, 1991. U.S. Patent 5,773,255 relates to glucose-responsive insulin secreting pancreatic beta cell lines. U.S. Patent 5,789,246 relates to hepatocyte precursor cells. Other progenitors of interest include but are not limited to chondrocytes, osteoblasts, retinal pigment epithelial cells, fibroblasts, skin cells such as keratinocytes, dendritic cells, hair follicle cells, renal duct epithelial cells, smooth and skeletal muscle cells, and vascular endothelial cells.

Scientists at Geron Corporation have discovered that culturing pPS cells or embryoid body cells in the presence of ligands that bind growth factor receptors promotes enrichment for neural precursor cells. The growth environment may contain a neural cell supportive extracellular matrix, such as fibronectin. Suitable growth factors include but are not limited to EGF, bFGF, PDGF, IGF-1, and antibodies to receptors for these ligands. The cultured cells may then be optionally separated based on whether they express a marker such as A2B5. Under the appropriate circumstances, populations of cells enriched for expression of the A2B5 marker may have the capacity to generate both neuronal cells (including mature neurons), and glial cells (including astrocytes and oligodendrocytes). Optionally, the cell populations are further differentiated, for example, by culturing in a medium containing an activator of cAMP. See International Patent Publication WO 01/81549 (Geron Corporation).

Scientists at Geron Corporation have discovered that culturing pPS cells or embryoid body cells in the presence of a hepatocyte differentiation agent promotes enrichment for hepatocyte-like cells. The growth environment may contain a hepatocyte supportive extracellular matrix, such as collagen or Matrigel®. Suitable differentiation agents include various isomers of butyrate and their analogs, exemplified by n-butyrate. The cultured cells are optionally cultured simultaneously or sequentially with a hepatocyte maturation factor, such as an organic solvent like dimethyl sulfoxide (DMSO); a maturation cofactor such as retinoic acid; or a cytokine or hormone such as a glucocorticoid, epidermal growth factor (EGF), insulin, TGF-α, TGF-β, fibroblast growth factor (FGF), heparin, hepatocyte growth factor (HGF), IL-1, IL-6, IGF-I, IGF-II, and HBGF-1. See International Patent Application PCT/US01/15861 (Geron Corporation).

Scientists at Geron Corporation have discovered that it is also possible to differentiate hPS cells into a highly enriched population comprising cardiomyocytes or cardiomyocyte precursors. The cardiomyocyte lineage cells can be obtained, for example, by differentiating hES cells in a growth environment comprising a cardiotrophic factor that affects DNA-methylation, exemplified by 5-azacytidine. Spontaneously contracting cells can then be separated from other cells in the population, for example, by density centrifugation. Further process steps can include culturing the cells in a medium containing creatine, carnitine, or taurine. Alternatively, it is possible to differentiate hPS cells into a highly enriched population comprising osteoprogenitors or osteoblasts expressing osteocalcin and collagen-1. The cells can be obtained by differentiating pPS cells in a medium containing a bone morphogenic protein (particularly BMP-4), a ligand for a human TGF-β receptor, or a ligand for a human vitamin D receptor.

Differentiated cells can be characterized by morphological features, detection or quantitation of expressed cell markers and enzymatic activity, and determination of the functional properties of the cells in vivo. Identifying markers for neural cells include β-tubulin III or neurofilament, characteristic of neurons; glial fibrillary acidic protein (GFAP), present in astrocytes; galactocerebroside (GalC) or myelin basic protein (MBP); characteristic of oligodendrocytes; OCT-4, characteristic of undifferentiated hES cells; nestin, characteristic of neural precursors and other cells. Glutamic acid decarboxylase or GABA identify GABA-secreting neurons; dopa decarboxylase, dopamine, or tyrosine hydroxylase identify dopaminergic neurons.

Markers for liver cells include α-fetoprotein (liver progenitors); albumin, α₁-antitrypsin, glucose-6-phosphatase, cytochrome p450 activity, transferrin, asialoglycoprotein receptor, and glycogen storage (hepatocytes); CK7, CK19, and γ-glutamyl transferase (bile epithelium). Cells in mixed cell populations can be identified using the following markers. For skeletal muscle: myoD, myogenin, and myf-5. For endothelial cells: PECAM (platelet endothelial cell adhesion molecule), Flk-1, tie-1, tie-2, vascular endothelial (VE) cadherin, MECA-32, and MEC-14.7. For smooth muscle cells: smooth muscle actin and specific myosin heavy chain. For cardiomyocytes: GATA-4, Nkx2.5, cardiac troponin I, α-myosin heavy chain, cardiac troponin T (cTnT), or atrial natriuretic factor (ANF). For pancreatic cells, pdx and insulin secretion.

### Differentiating pPS cells into Cells that Induce Immunotolerance

Human ES cells can be differentiated into tolerizing cells by forming embryoid bodies as described or by direct differentiation in a suitable culture environment with suitable medium.

In a typical procedure, the cells are cultured as aggregates or monolayers, in liquid suspension or in semi-solid media such as methylcellulose or agarose. Growth factors are typically added 1-2 weeks after differentiation begins. Outgrowth of various populations of hematopoietic cells can be facilitated using IL-3, vascular endothelial growth factor (VEGF), thrombopoietin (Kit ligand), IL-1, IL-6, IL-11, M-CSF, or GM-CSF. Possible adjuncts include stem cell factor, IL-2, IL-7, insulin-like growth factor 1, erythropoietin, basic fibroblast growth factor, endothelial cell growth supplement, G-CSF, Flt-3 ligand, anti-M-CSF, and anti-TGF-β. Candidate costimulatory molecules include hydrocortisone, dexamethazone, Con A, PHA, and LPS.

In some instances, the culture environment may include feeder cells, especially mouse or human derived bone marrow stromal cells (e.g. S17, RP.0.10, ST2, PA6, Ac6 or freshly isolated primary cultures). Other possible feeder cells include fetal liver stromal cells (e.g. FLS4.1), yolk sac cells (e.g., C166), thymic stromal cells, activated spleen cells, or endothelial cells. Alternatively, the cells can be grown on an extracellular matrix, such as Matrigel®, laminin, fibronectin or collagen, or matrixes produced by feeder cells. Without feeder cells being present, some of the activity they provide can be replaced by using conditioned medium (e.g., supernatant from stromal cells). To promote hematopoiesis, the cells may be cultured in normoxic conditions (19% O₂) or In low-oxygen (5% O₂) e.g. in incubators with adjustable oxygen content. The choice of particular growth conditions depends partly on the mechanics of culture and the cell subpopulation that is desired.

The cells are cultured for sufficient time until colonies form with a cobblestone-like appearance. The colonies can then be passaged and tested for phenotypic markers by flow cytometry, immunohistochemistry, or enzyme-linked immunoassay. Expression can also be detected at the mRNA level by reverse transcriptase-PCR using marker-specific primers (Moore, Clin. Cancer Res. 1:3, 1995).

Relevant markers are as follows: For human hematopoietic precursors or stem cells: CD34 +, CD38 -, Thy+, HLA-DR-, CD45RO +, CD71 lo, Rhodamine 123 lo, GATA-1, AC133, β-major globulin, β-major globulin like gene βH1. For mesenchymal stem cells: CTLA-4, SH2 +, SH3 +, CD29 +, CD44 +, CD71 +, CD90 +, CD106 +, CD14 -, CD34 -, CD45 -. For lymphoid cells: CD45 +. For T cells: CD2 +, CD3 +, CD4 +, CD8 +, T cell receptors, IL-2 receptor. For B cells: HLA Class II, CD19 +, lg gene rearrangement. For dendritic cells: DEC-205 +, CMRF-44 +, CMRF-56 +, S100 +. For natural killer cells: CD16 +,CD2 +, CD3 -. For macrophage/monocytes: HLA Class II, CD14 +, CD15 +. For megakaryocytes: CD41b +. For erythroid cells: glycophorin A +, hemoglobin.

Hematopoietic progenitors can be assayed for colony formation by plating cells into methylcellulose containing factors such as IL-1, IL-3, KL, G-CSF, GM-CSF, M-CSF, and EPO, and then enumerating the number and type of colonies formed (e.g. HPP-CFC, CFU-GM, BFU-E). The cells can also be plated onto allogeneic bone marrow stromal cells and the long-term proliferative potential evaluated by the number and size of colonies generated and the phenotype of the cells in the colonies.

Differentiation potential of hematopoietic cells can be assessed in animal models for their ability to form colonies in the spleen (CFU-S). Differentiated cells are injected intravenously into the animals and the formation of colonies in the spleen is enumerated after about 2 weeks. They can also be assessed for their ability to repopulate the hematopoietic system of sub-lethally irradiated mice or to rescue lethally irradiated mice. Cells are injected intravenously and engraftment is monitored by analyzing the percentage of human myeloid or lymphoid cells in the mouse blood using human specific antibodies in FACS analysis. Suitable markers include CD3 (T cells), CD19 (B cells) and CD14/15 (myeloid cells). Sometimes whole bone marrow is co-injected to help maintain survival, and the two donor populations are distinguished by their MHC type.

Optionally, tolerizing cells can be separated from differentiated cells of other lineage in the culture, or particular cell subsets can be separated using antibody specific for the markers listed above. For example, cells can be enriched by fluorescence-activated cell sorting, or immunomagnetic bead sorting, for the phenotype CD34 +, CD38 -, CD34 +, and Thy +. A variation of this technique is to use a promoter-reporter construct which marks the desired cell type for selection. For example, the CD34 promoter or enhancer (Burn et al., Blood 80:3051, 1992; Radomska et al., Gene 222:305, 1998; GenBank Accession No. AF047373) can drive expression of an encoding region for a drug resistance gene, or a fluorescent label such as green fluorescent protein (U.S. Patent 6,166,178, Geron Corp.). Transient expression of the promoter-reporter (for example, using an adenovirus vector) in a mixed cell population permits CD34 + cells to be selected out by culturing in the presence of the corresponding antibiotic, or by fluorescence-activated cell sorting, respectively.

In the course of preparing cell populations suitable for inducing immunotolerance for use in this invention, the practitioner can optionally employ adjunct methods described elsewhere.

For example, WO 93/18137 (SyStemix) advocates culturing hematopoietic stem cells for 12 h in a medium comprising at least 10 ng/mL leukemia inhibitory factor (LIF). U.S. Patent 5,635,387 (CellPro) outlines methods and a device for culturing human hematopoietic cells and their precursors, particularly CD34 positive cells, using a nutrient medium containing growth factors. U.S. Patent 5,733,541 outlines a process for propagating and maintaining hematopoietic precursors that are CD34 +ve, HLA-DR +ve, Thy-1 +ve, and Lin -ve. U.S. Patent 6,015,554 (SyStemix) relates to methods for obtaining hematopoetic cell precursors enriched for progenitors that are CD34 +ve, CD45RA +ve, and CD10 +ve.

Keller et al. (Curr. Opin. Cell Biol. 7:862, 1995; Mol. Cell Biol. 13:473, 1993; Development 125:725, 1998) outline a two-step differentiation protocol In which embryoid bodies are formed from mouse ES cells, dissociated, and then replated into semi-solid medium (1% methylcellulose) or liquid cultures containing different growth factor combinations.

Kaufman et al. (Keystone Symposium on Stem Cells, 2000, abstract 315) cultured human ES cells on mouse bone marrow stromal cell line S17 or mouse yolk sac cell line C166 without exogenously added growth factors. Cobblestone colonies were observed after -7 days; at 14-21 days some cells stained positively for CD34 but not for CD45.

Nakano et al., Science, 1994 cocultured mouse ES cells with an M-CSF deficient stromal cell line OP9. Potocnik et al. (EMBO J. 13:5274, 1994) cultured mouse ES cells as embryoid bodies in either liquid medium or semi-solid methylcellulose in a low oxygen (5% O₂) atmosphere without additional exogenous factors. Palacios et al. (Proc. Natl. Acad. Sci. USA 89:9171, 1992; Proc. Natl. Acad. Sci. USA 92:7530, 1995) plated mouse ES cells onto inactivated stromal cells in fetal calf serum with growth factors such as IL-3, IL-6, IL-7 or fetal liver stromal cell conditioned medium.

Fairchild et al. (Curr. Biol. 10:1515, 2000) reported establishment of long-term cultures of immature dendritic cells from mouse embryonic stem cells. The DC's shared many characteristics with macrophages, but upon maturation, they acquired the allostimulatory capacity and surface phenotype of classical DC's, including expression of CD11c, MHC class II, and costimulatory molecules. Prospects of DC's for transplantation tolerance is reviewed by Fairchild et al. in Curr. Opin. Immunol. 12:528, 2000. Dendritic cells capable of inducing tolerance may be generated in some circumstances by culturing in medium containing GM-CSF and IL-4, and then with low-level GM-CSF and IL-10.

In some instances, the tolerizing cells are kept as a bank to tolerize patients on demand for regenerative tissue from the same line. To improve replicative capacity of the cells and facilitate banking, they can be telomerized by transfection or transduction with a suitable vector, homologous recombination, or other appropriate technique, so that they express the telomerase catalytic component (TERT). Particularly suitable is the catalytic component of human telomerase (hTERT), provided in International Patent Publication WO 98/14592. Transfection and expression of telomerase in human cells is described in Bodnar et al., Science 279:349, 1998 and Jiang et al., Nat. Genet. 21:111, 1999.

Before and after telomerization, telomerase activity and expression of hTERT gene product can be determined using commercially available reagents and established methods. For example, pPS cells are evaluated for telomerase using TRAP activity assay (Kim et al., Science 266:2011, 1997; Weinrich et al., Nature Genetics 17:498, 1997). The following assay kits are available commercially for research purposes: TRAPeze® XL Telomerase Detection Kit (Cat. s7707; Intergen Co., Purchase NY); and Telo*TAGGG* Telomerase PCR ELISAplus (Cat. 2,013,89; Roche Diagnostics, Indianapolis IN). hTERT expression can also be evaluated at the mRNA by RT-PCR. The following assay kit is available commercially for research purposes: LightCycler TeloTAGGG hTERT quantification kit (Cat. 3,012,344; Roche Diagnostics).

For therapeutic use, it is desirable that tolerizing cell populations of this invention be substantially free of undifferentiated pPS cells. One way of depleting undifferentiated stem cells from the population is to transfect them with a vector in which an effector gene under control of a promoter that causes preferential expression in undifferentiated cells. Suitable promoters include the TERT promoter and the OCT-4 promoter. The effector gene may be directly lytic to the cell, encoding, for example, a toxin, or a mediator of apoptosis. Exemplary apoptosis genes are the caspase family (Shinoura et al., Cancer Gene Ther. 7:739, 2000; Koga et al., Hum. Gene Ther. 11:1397, 2000). Optionally, the effector gene can be further linked to a molecular switch (such as a tetracycline resistance element, Gossen et al., Curr. Opin. Biotechnol. 5:516, 1994; U.S. Patent 5,464,758; Clackson, Curr. Opin. Chem. Biol. 1:210, 1997) that causes killing of the undesired cells only in the presence of the inducing drug (tetracycline). Alternatively, the effector gene may have the effect of rendering the cell susceptible to toxic effects of an external agent, such as an antibody or a prodrug. Exemplary is a herpes simplex thymidine kinase (*tk*) gene, which causes cells in which it is expressed to be susceptible to ganciclovir. Suitable pTERT-*tk* constructs are provided in WO 98/14593 (Morin et al.).

### Using Two Matched Cell Populations in Tissue Regeneration

To reconstitute cellular function in an individual, a first cell population is administered that has been differentiated from human pluripotent stem (hPS) cells into a phenotype that renders the individual immunotolerant to the HLA tissue type of the tolerizing cell population. The tissue regeneration allograft (matched with the tolerizing cells) can be administered or implanted simultaneously, but more typically is administered a few weeks later.

This invention provides animal models for evaluating the viability of tolerizing protocols. The first is a mouse model, using mouse ES cells prepared according to established methods (supra). Mouse ES cells are prepared according to standard methods from inbred BALB/c, C3H, or C57BL strains. They are differentiated into tolerizing cells as described in the previous section. The tolerizing cells are then injected intraportally or through the tail vein into mice of another inbred strain with a different H-2 type. Primary testing range is between 10⁶ and 10⁷ cells per mouse. In parallel experiments, some animals receive a second dose of tolerizing cells ~5 days after the first.

A week after the first tolerizing treatment, a full-thickness skin allograft is harvested from the dorsal wall of the same donor strain, and depilated. It is then sutured into the right thoracic wall of the recipient animal using 6-0 nylon. Over the course of the next 6 weeks or more, the graft is inspected to determine whether normal epithelium remains in the graft beds. Without prior tolerization, skin grafts are normally rejected within ~2 weeks.

Blood is sampled before the skin allograft is performed, and then once every two weeks after transplant. A number of assays can be performed. Alloreactive antibody is measured by mixing recipient serum with fresh complement and ⁵¹Cr-labeled target cells of the donor strain (such as cultured fibroblasts, or cells differentiated from the ES line). Alloreactive cytotoxic T cells are measured by combining the ⁵¹Cr-labeled target cells with Ficoll®-separated peripheral blood mononuclear cells from the recipient. T cell helper/inducers are measured by combining recipient PBMC with irradiated donor PBMC or spleen cells, and measuring [³H]thymidine incorporation. Supernatant from the mixed lymphocyte reaction can also be measured for cytokine secretion by sandwich ELISA for IFNγ, IL-2, TNFα, or IL-10 (antibody available from Genzyme) as a measure of cellular immunoreactivity.

Grafts are inspected every few days for signs of rejection such as hair loss, necrosis, and absence of normal epithelium in the graft beds. Animals with surviving grafts can be tested at -6 weeks for the extent and specificity of immune tolerance by suturing in a second graft on the opposite flank, from the same donor strain or a third-party donor. Chimerism of the recipients is evaluated by FACS analysis by harvesting spleen or bone marrow cells and staining with specific antibody to the donor H-2 allotype, recipient H-2 allotype, and hematopoietic markers such as CD4, CDB, B220, and Mac-1, Graft acceptance is expected to correlate with lower alloresponse in the immunological assays, and may also correlate with evidence of chimerism.

The second model is a primate model, using rhesus ES cells (Thomson et al., Proc. Natl. Acad. Sci. USA 92:7844, 1995), or human ES cells (Thomson et al., Science 282:114, 1998). The ES cells are differentiated into toleragenic cells as described above, and injected intravenously into rhesus monkeys, scaling up appropriately the optimal dose determined from the mouse model. The animals may receive a second dose about 1 week later. Simultaneously, the same ES cell line is differentiated into hepatocyte-like cells or neuronal cells as described earlier.

At about the 2 week point, the tolerized animals receive the allograft of replacement tissue. Hepatocyte-like cells and neural precursors are made from the same ES cell line as described earlier. The differentiated cells are labeled intrinsically with BrdU or with an expression vector encoding a reporter gene such as green-fluorescent protein (GFP). Hepatocyte-like cells are implanted into the kidney capsule or into the spleen. Blood is collected periodically and assayed for signs of an immune response to alloantigen, and also for chimerism, as in the mouse model. After 2, 4, or 6 weeks, a biopsy sample is taken from the implant site. Biopsy samples are examined for evidence of surviving graft cells by measuring the intrinsic label, and Immunohistochemistry for liver-specific, MHC specific, or human-specific markers (if the ES line was of human origin).

Upon determination of suitable conditions for inducing allo-specific immune tolerance, additional experiments can be undertaken to evaluate the efficacy of the tissue regeneration protocol using accepted animal models. For example, the efficacy of neural cell transplants can be assessed in a rat model for acutely injured spinal chord as described by McDonald et al. (Nat. Med. 5:1410, 1999). A successful transplant will show transplant-derived cells present in the lesion 2-5 weeks later, migrating along the cord from the lesioned end, accompanied by an improvement in the animal's gate, coordination, and weight-bearing. Hepatocyte replacement can be assessed in animal models for ability to repair liver damage. One such example is damage caused by intraperitoneal injection of D-galactosamine (Dabeva et al., Am. J. Pathol. 143:1606, 1993). The efficacy of cardiomyocytes prepared according to this invention can be assessed in animal models for cardiac cryoinjury, which causes 55% of the left ventricular wall tissue to become scar tissue without treatment (Li et al., Ann. Thorac. Surg. 62:654, 1996; Sakai et al., Ann. Thorac. Surg. 8:2074, 1999, Sakai et al., J. Thorac. Cardiovasc. Surg. 118:715, 1999).

In using this invention to induce immunotolerance in a subject about to receive an allograft, the practitioner can optionally employ adjunct techniques described elsewhere.

For example, WO 99/51275 (Osiris Therapeutics) proposes to use mesenchymal stem cells presenting membrane-bound antigen to induce specific T cell anergy, thereby inducing immunosuppression. WO 93/13785 (Sachs et al.), WO 95/21527 (Sachs et al.), WO 97/41863 (Sytes et al.), and U.S. Patent 6,006,752 (Sytes et al.) propose methods for inducing immunotolerance, in which hematopoietic stem cells of a donor animal of one species are administered to a recipient of a second species. This forms mixed chimerism in the recipient, which allows them to receive a graft from the first species.

U.S. Patent 5,843,425 (Sachs et al.) explains how T cells present in a tolerizing hematopoietic cell preparation can be depleted using specific antibody. WO 99/39727 (Sytes et al.) advocates administering the hematopoietic cells in combination with something that inhibits CD40 from interacting with its ligand. According to U.S. Patent 5,876,708 (Sachs et al.), the tolerizing effect of the hematopoietic cells can be supplemented by inactivating T cells in the recipient (e.g., using anti-CD4 or anti-CD8), and administering an immunosuppressive agent (such as cyclosporin A).

U.S. Patents 5,858,963, U.S. Patent 5,863,528, and WO 97/41863 (Sachs et al.) outline how tolerance can be induced in an animal model using bone marrow cells in combination with cytokines such as stem cell factor, IL-3, GM-CSF, and IL-10. WO 93/09815 (Sachs et al.) proposes transfecting bone marrow hematopoietic cells with nucleic acid encoding MHC antigen to confer tolerance to a transplanted tissue in a recipient animal. WO 95/03062 (CellPro) suggests tolerizing a recipient for solid organ transplantation by harvesting cells from the organ donor, enriching for hematopoietic cells (such as CD +ve cells), and infusing them into the recipient before transplant.

Morita et al. (Proc. Natl. Acad. Sci. USA 95:6947, 1998) outline a strategy for inducing tolerance for organ allografts. Recipient mice were injected into the portal vein with spleen cells from an allogeneic donor, and given a skin graft from the same donor a week later. In some animals, the grafts survived a year after transplantation, which was accompanied by established microchimerism. Donor T cells in the administered composition apparently facilitated engraftment, cytotoxic T lymphocytes inducing donor specific anergy. An intravenous dose of bone marrow cells from the same source 5 days later significantly enhanced tolerance induction.

Starzl et al. (Lancet 339:1579, 1992; N. Engl. J. Med. 328:745, 1993) observed that human patients who accept liver transplants have donor-derived dendritic cells and macrophages that migrate from the allograft into recipient lymph nodes. Other publications relating to inducing chimerism and immune tolerance are listed below.

A human patient can be treated according to this invention by administering a first cell population differentiated from human pluripotent stem (hPS) cells into a phenotype that renders the individual immunotolerant to a second cell population, as described earlier. Intravasular administration is currently the preferred route, although other routes are contemplated (such as intrasplenic injection). The predicted dose is a cell suspension in which between ~10⁹ and 10¹¹ cells have toleragenic potential. If necessary, the patient can be treated with an ablative or partly ablative dose of γ-irradiation or chemotherapy to create a hematopoietic space and allow chimerism with the engrafting cells to take place.

The patient can be monitored for the establishment of immune tolerance by harvesting PBMC, and conducting a one-way mixed lymphocyte reaction using irradiated Class-II presenting hPS donor cells (using acridine orange or cytokine secretion for rapid read-out). Additional dose cycles are given as needed.

After sufficient time and treatment for tolerance to take effect, the patient is administered with regenerative tissue autogeneic with (or HLA matched with) the tolerizing cells. Depending on the degree of allotolerance, it may be beneficial to maintain the patient on immunosuppressive drugs (such as cyclosporin A or anti-CD4 antibody) until the graft takes, migrates, and assumes its functional role. In instances where it is not possible to pre-tolerize the patient, it may still be beneficial to administer the tolerizing cells simultaneously or sequentially with the regenerative cells. Ultimate choice of the treatment protocol, dose, and monitoring is the responsibility of the managing clinician.

Cells useful for inducing specific immune tolerance according to this invention is optimally supplied in a pharmaceutical composition, comprising an isotonic excipient prepared under sufficiently sterile conditions for human administration. For general principles in medicinal formulation, the reader is referred to Cell Therapy: Stem Cell Transplantation, Gene Therapy, and Cellular Immunotherapy, by G. Morstyn & W. Sheridan eds, Cambridge University Press, 1996; and Hematopoietic Stem Cell Therapy, E.D. Ball, J. Lister & P. Law, Churchill Livingstone, 2000.

The toleragenic composition may be packaged with written instructions for use of the cells in inducing tolerance. The hematopoietic cells are usually matched with HLA compatible tissue that will be used for tissue regeneration, and the two compositions can be shipped together in kit form.

*It will be recognized that the compositions and procedures provided in the description can be effectively modified by those skilled in the art without departing from the spirit of the invention embodied in the claims that follow.*

## Claims

1. A method for preparing cells for therapeutic use, comprising:
a) differentiating human pluripotent stem (hPS) cells into a first cell population,
wherein the first cell population has characteristics of dendritic cells;
b) differentiating human pluripotent stem (hPS) cells into a second cell population;
wherein the first cell population is MHC compatible with the second cell population, and whereupon administration of the first population to an individual renders the individual immunotolerant to the second cell population.

2. A method of reconstituting cellular function in an individual, comprising:
a) administering to the individual the first cell population of claim 1, thereby rendering the individual immunotolerant to the second cell population; and
b) administering to the individual the second cell population of claim 1, thereby reconstituting the cellular function.

3. The method of any preceding claim, wherein the first cell population and the second cell population are differentiated from the same hPS cells or their progeny.

4. The method of any preceding claim, wherein the second cell population comprises one of the following cell types or their lineage-restricted precursors: hepatocytes, neurons, oligodendrocytes, astrocytes, cardiomyocytes, chondrocytes, osteogenic cells, pancreatic islet cells, endothelial cells, skeletal muscle cells, or skin cells.

5. A combination of pharmaceutical compounds, comprising in separate containers:
a) a first cell population that has been differentiated from human pluripotent stem (hPS) cells into a phenotype that renders a subject to whom it is administered immunotolerant to a second cell population, wherein the first cell population has characteristics of dendritic cells; and
b) the second cell population that is MHC compatible with the first cell population.

6. The pharmaceutical compounds of claim 5 wherein the first cell population and the second cell population are differentiated from the same hPS cell line.

7. The pharmaceutical compounds of claim 5 or claim 6, wherein the second cell population comprises one of the following cell types or their lineage-restricted precursors: hepatocytes, neurons, oligodendrocytes, astrocytes, cardiomyocytes, chondrocytes, osteogenic ells, pancreatic islet cells, endothelial cells, skeletal muscle cells, or skin cells.

8. Use of a first cell population differentiated from hPS cells into a phenotype that renders an individual to whom they are administered immunotolerant to a second cell population, in the preparation of a medicament for treatment of the human or animal body by surgery or therapy, wherein the first cell population has characteristics of dendritic cells.

9. Use of a first cell population differentiated from hPS cells into a phenotype that renders an individual to whom they are administered immunotolerant to a second cell population, in the preparation of a medicament for rendering a human or animal body immunotolerant to the second cell population, wherein the first cell population has characteristics of dendritic cells.

10. Use of a first cell population and a second cell population that are autogeneic, wherein the second population is differentiated from hPS cells into a phenotype that reconstitutes cellular function in an individual, and the first population renders the individual immunotolerant to the second cell population, each for the preparation of a medicament for simultaneous or sequential administration to the human or animal body, wherein the first cell population has characteristics of dendritic cells.

11. The use according to any one of claims 8 to 10, wherein the second cell population comprises one of the following cell types or their lineage-restricted precursors: hepatocytes, neurons, oligodendrocytes, astrocytes, cardiomyocytes, chondrocytes, osteogenic ells, pancreatic islet cells, endothelial cells, skeletal muscle cells, or skin cells.

12. The use according to any one of claims 8 to 11 wherein the first cell population is formulated for administration into the circulation.
